# EUROPÄISCHE PATENTANMELDUNG

(11) **EP 3 695 863 A1**
(43) Veröffentlichungstag der Anmeldung: **19.08.2020**
(21) Anmeldenummer: 19157362.5
(22) Anmeldetag: 15.02.2019
(51) Int. Cl.: A61M 5/20, A61M 5/24, A61M 5/32

(54) **MODULARER SPRITZENHALTER UND SPRITZENMONTAGEVERFAHREN**

(71) Anmelder: TecPharma Licensing AG, 3401 Burgdorf (CH)
(72) Erfinder: Schrul, Christian, 3400 Burgdorf (CH); Tschirren, Markus, 3400 Burgdorf (CH)

(57) **Zusammenfassung**

Die Erfindung betrifft eine Verwendung von kleinvolumigen Produktbehältern in Injektionsgeräten für nominal grossvolumige Produktbehälter. Erfindungsgemäss umfasst ein modularer Spritzenhalter einen Adapter (14) und einen von einem Gehäuse des Injektionsgeräts verschiedenen Adapterhalter (15) zum Halten einer Fertigspritze in einer Spritzeneinheit des Injektionsgeräts. Der Adapter weist einen hohlzylindrischen, starren Adapterkörper (14c) auf und ein daran beweglich angebrachtes und zumindest radial auslenkbares Auflageelement (14a) für eine Spritzenschulter der Fertigspritze. Der Adapter ist ausgebildet zur Einführung der Fertigspritze unter Auslenkung des Auflageelements durch eine Nadelschutzkappe der Fertigspritze. Der Adapterhalter ist ausgebildet zur Aufnahme des Adapters, so dass dadurch das Auflageelement von einem Halteabschnitt (15b) des Adapterhalters gegen ein radiales Auslenken oder Ausscheren in einer Halteposition in Eingriff mit der Spritzenschulter blockiert wird. Ein flexibles Schulterauflageelement zur Ableitung von axialen Kräften eines Produktbehälters wird also nach Aufnahme von Adapter und Produktbehälter im Adapterhalter durch den Adapterhalter selbst gegen laterale Auslenkung gesichert.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf eine Verwendung von kleinvolumigen Produktbehältern in Injektionsgeräten für nominal grossvolumige Produktbehälter.

### HINTERGRUND DER ERFINDUNG

Injektionsgeräte oder Injektionsvorrichtungen zum vereinfachten Verabreichen einer Substanz umfassen unter anderem so genannte Autoinjektoren, welche ein Energiespeicher- oder Antriebselement aufweisen, mit welchem die Ausschüttung automatisch, das heisst ohne extern von einem Benutzer zuzuführende oder aufzuwendende Kraft, durchgeführt werden kann. Das Energiespeicher- oder Antriebselement speichert die für eine automatische Substanzabgabe erforderliche Energie vorteilhaft in mechanischer Form. Ein solches Energiespeicher- oder Antriebselement kann eine Feder sein, welche in einem gespannten Zustand in das Injektionsgerät eingebaut wird und durch Entspannen Energie abgibt. Die Energieabgabe erfolgt an eine Kolbenstange oder ein Druckelement, welches einen Kolben in einen Produktbehälter einschiebt. Das Energiespeicher- oder Antriebselement kann auch vorgesehen sein um den Vorgang des Einstechens einer Injektionsnadel zu automatisieren. Alternativ kann zu diesem Zweck ein weiteres separates Antriebselement vorgesehen sein, oder der Einstechvorgang erfolgt manuell, also ausschliesslich durch einen Benutzer, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter radial, axial und vorzugsweise auch drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule zur wiederholt lösbaren Verbindung mit Einweg-Injektionsnadeln oder eine Einweg-Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben. Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung WO2016/205963 beschreibt einen beispielhaften Autoinjektor, umfassend ein Gehäuse mit Längsachse, eine Auslösevorrichtung, einen axial fest im Gehäuse angeordneten Produktbehälter. Der Autoinjektor umfasst weiter eine in einer Längsrichtung zwischen einer proximalen und einer distalen Position verschiebbare Nadelschutzhülse, welche mit einer Nadelschutzhülsenfeder als separatem Antriebselement gekoppelt ist. Eine erste Rückkopplungsvorrichtung mit einem durch die Ausschüttfeder beschleunigten ersten Anschlagelement signalisiert den Beginn der Substanzabgabe. Eine zweite Rückkopplungsvorrichtung mit einem durch die Nadelschutzhülsenfeder zu einem Anschlag hin beschleunigten zweiten Anschlagelement dient zur Erzeugung eines akustischen Signals nach Abgabe einer bestimmten Menge an Substanz. Eine Spiral- oder Triebfeder, in welcher Energie für das automatische Ausschütten von Produkt gespeichert werden kann, ist mit der Auslösevorrichtung gekoppelt, wobei ein erstes Ende der Spiralfeder mit dem Gehäuse verbunden ist, und ein zweites Ende der Spiralfeder rotationsfest mit einem koaxial zur Längsachse angeordneten Rotationsglied in Form einer Gewindestange verbunden ist. Die Gewindestange greift über ein Gewinde in ein im Gehäuse nicht rotierendes Vortriebsglied in Form einer hülsenförmigen Kolbenstange, welche bei einer Verschiebung in distale Richtung den Stopfen des Produktbehälters mit einer zumindest annähernd konstanten Ausschüttgeschwindigkeit mitbewegt. Der Autoinjektor ist für Fertigspritzen umfassend einen Produktbehälter mit einer vorgegebenen Grösse und einem nominalen Füllvolumen von 2.25 ml ausgelegt, wobei die Spiralfeder auch für ein auszuschüttendes Produkt mit einer hohen Viskosität von mindestens 5, bevorzugt mindestens 15 cP (0.015 kgm⁻¹s⁻¹) geeignet ist. Die Fertigspritze umfasst weiter eine Nadel, welche vor Gebrauch von einem elastischen Nadelschutzelement und einer festen Nadelschutzkappe oder Rigid Needle Shield RNS zur Gewährleistung der Sterilität und Unversehrtheit umgeben ist.

Die Patentanmeldung EP 2968063 A1 beschreibt einen Produktbehälterhalter oder Medikamentenbehälterträger für einen ersten Produkt- oder Medikamentenbehälter mit einer ersten vorgegebenen Grösse, der in der Lage ist, mehr als ein erstes Volumen eines Medikaments zu enthalten. Der Behälterträger umfasst einen Körper und einen mit dem Körper verbundenen Adapter, wobei der Adapter ausgebildet ist, einen zweiten Medikamentenbehälter mit einer zweiten vorgegebenen Grösse zu tragen, der in der Lage ist, nicht mehr als das erste Volumen des Medikaments zu enthalten. Der zweite Medikamentenbehälter wird axial und drehfest in dem Adapter fixiert, und der Adapter ist wiederum axial und drehfest in dem Behälterträger gehalten. Dazu umfasst der Adapter einen Arm mit einer ersten und zweiten Anlagefläche, welche mit einem proximalen Flansch des zweiten Medikamentenbehälters in Eingriff kommen. Der Adapter weist eine umlaufende, nichtflexible distale Rippe oder Auflagefläche auf, an welche ein distales Ende oder eine Schulter des zweiten Medikamentenbehälters anliegt, und welche eine axiale, über den Stopfen auf den Medikamentenbehälter wirkende Kraft aufnimmt.

Die Patentanmeldung WO 2012/164389 beschreibt in Fig.13 bis 15 einen typähnlichen Adapter mit flexiblen Fingern, welche distal an einer Hülse angeordnet sind und an ihrem Ende nach proximal gerichtete Auflageflächen für eine Produktbehälterschulter aufweisen. Bei der Montage einer Fertigspritze wird deren Nadelschutzkappe durch eine mittels der Finger gebildete Öffnung hindurchgepresst wobei die Finger radial nach aussen ausgelenkt werden. Anschliessend wird ein Sicherungsring nach vorne geschoben und dadurch die Finger wieder nach innen gelenkt und die Auflageflächen in einer Halteposition in Eingriff mit der Produktbehälterschulter blockiert. Dadurch kann die durch die Innenkanten der Auflageflächen gebildete Öffnung im blockierten Zustand einen Durchmesser aufweisen, welcher kleiner ist als ein Aussendurchmesser der Nadelschutzkappe. Die Auflageflächen kommen somit zumindest teilweise zwischen Nadelschutzkappe und Produktbehälterschulter zu liegen, und ermöglichen auch eine Aufnahme von axialen Kräften der Produktbehälterschulter falls deren Aussendurchmesser nur unwesentlich oder gar nicht grösser ist als der Durchmesser der Nadelschutzkappe.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektionssystem" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist Aufgabe der Erfindung, die Verwendung von Produktbehältern mit einem kleinen Füllvolumen in einem Injektionsgerät für Produktbehälter mit einem grossen Füllvolumen zu vereinfachen. Die Aufgabe wird gelöst durch einen modularen Spritzenhalter und durch ein Verfahren zur Montage eines Injektionsgeräts mit den Merkmalen der unabhängigen Ansprüche. Bevorzugte Ausführungsformen der Erfindung sind Gegenstand der abhängigen Ansprüche.

Erfindungsgemäss umfasst ein modularer Spritzenhalter einen Adapter und einen Adapterhalter zum Halten einer Fertigspritze in einer Spritzeneinheit eines Injektionsgeräts mit einem Gehäuse, welches von einem Benutzer greifbar ist und eine Längsachse definiert. Der Adapterhalter kann in das Gehäuse eingeführt und im Gehäuse axial und drehfest gehalten werden. Der Adapter weist einen hohlzylindrischen, starren Adapterkörper auf und ein daran beweglich angebrachtes und zumindest radial auslenkbares Auflageelement für eine Spritzenschulter der Fertigspritze. Der Adapter ist ausgebildet oder vorbereitet zur Einführung der Fertigspritze in Richtung der Längsachse unter Auslenkung des Auflageelements durch eine Nadelschutzkappe der Fertigspritze. Der Adapterhalter ist ausgebildet oder vorbereitet zur Aufnahme des Adapters, so dass dadurch das Auflageelement von einem Halteabschnitt des Adapterhalters gegen ein radiales Auslenken oder Ausscheren in einer Halteposition in Eingriff mit der Spritzenschulter blockiert wird.

Ein flexibles Schulterauflageelement zur Ableitung von axialen Kräften eines Produktbehälters wird also nach Aufnahme von Adapter und Produktbehälter im Adapterhalter durch den Adapterhalter selbst gegen laterale Auslenkung gesichert, ohne dass dazu eine manuelle Verschiebung eines separaten Sicherungsrings in distale Richtung notwendig wäre. Ein entsprechendes Montageverfahren reduziert sich also auf die Schritte
- Einsetzen des Adapterhalters in die Spritzeneinheit,
- Einführen der Fertigspritze in den Adapter, und
- Einführen oder Einschieben des Adapters mit der Fertigspritze in die Spritzeneinheit.

In einer bevorzugten Ausführungsform weist der Adapter des modularen Spritzenhalters einen flexiblen Finger mit einem zumindest annähernd konstanten Querschnitt auf, welcher am Adapterkörper befestigt ist und an seinem distalen Ende das Auflageelement zwecks Aufnahme von distalen Axialkräften der Spritzenschulter einer montierten Fertigspritze trägt. In Rotationsrichtung um die Längsachse L ist die Ausdehnung des Auflageelements grösser als die entsprechende Ausdehnung oder Breite des Fingers, das Auflageelement steht zumindest in eine und bevorzugt in beide Rotationsrichtungen vom Finger ab. Das Auflageelement umfasst einen Abschnitt oder einen Sektor eines Kreisringes mit einem mittleren Durchmesser entsprechend einem Durchmesser der Spritzenschulter, die dazugehörige Bogenlänge übertrifft also die Breite des Fingers. Durch diese Kombination werden eine maximale Auflagefläche des Auflageelements und eine klar definierte Beweglichkeit des Fingers gleichzeitig erreicht.

In einer vorteilhaften Ausführungsform sind der Adapter und der Adapterhalter derart ausgebildet, dass im montierten Zustand des Spritzenhalters im Injektionsgerät das Auflageelement in axialer Richtung unmittelbar an einer nach proximal orientierten Fläche des Gehäuses ansteht. Axiale Kräfte werden also von der Spritzenschulter an das Auflageelement und von diesem direkt an das Gehäuse weitergeleitet. Der Adapterhalter weist dazu bevorzugt am distalen Ende einen minimalen Innendurchmesser auf welcher grösser ist als ein Aussendurchmesser des Adapters beziehungsweise des Auflageelements.

In einer weiteren vorteilhaften Ausführungsform sind der Adapter und der Adapterhalter derart ausgebildet, dass im montierten Zustand des Spritzenhalters im Injektionsgerät die Fertigspritze um die Längsachse rotieren kann. Die Fertigspritze und insbesondere ein Fingerflansch der Spritze werden also weder durch das Gehäuse noch durch den rotationsfest im Gehäuse montierten Adapterhalter an einer Rotation gehindert. Bevorzugt ist der Adapter selbst rotationsfest im Adapterhalter aufgenommen und an seinem proximalen Ende ausreichend ausgeweitet um eine Rotation der Fertigspritze zu erlauben. Eine im Injektionsgerät frei drehbare Spritze gibt eine Rotation des Injektionsgerätes nicht an die Injektionsstelle weiter und verursacht dadurch weniger Schmerz beim Patienten. Eine zu diesem Zweck vorgesehene seitliche Ausweitung oder Verbreiterung von Adapter oder Adapterhalter kann zusätzlich auch als stirnseitige Auflage im Gehäuse dienen.

In einer bevorzugten Ausführungsform weist der Adapter des modularen Spritzenhalters mindestens zwei flexible Finger auf, welche am Adapterkörper befestigt sind und an ihren distalen Enden jeweils ein Auflageelement tragen zur Aufnahme von distalen Axialkräften der Spritzenschulter einer montierten Fertigspritze. Alternativ kann das Auflageelement einen radial nach innen gerichteten flexiblen Kragen umfassen, welcher durch zumindest einen Schlitz in radialer Richtung unterbrochen ist und durch die Nadelschutzkappe temporär ausgeweitet werden kann. Auf jeden Fall ist ein Durchmesser einer durch das oder die Auflageelemente gebildeten distalen Öffnung des Adapters kleiner als der maximale Aussendurchmesser der Nadelschutzkappe der Fertigspritze. Zudem ist eine Ausdehnung des Auflageelements in axialer Richtung an jedem Auflagepunkt kleiner als ein axialer Abstand zwischen einem proximalen Ende der Nadelschutzkappe und dem Auflagepunkt der Spritzenschulter. Dadurch ist bei einer satt aufliegenden und nach distal gehaltenen Spritze die Nadelschutzkappe von den Auflageelementen unbelastet, und ein unbeabsichtigtes, die Sterilität der Nadel gefährdendes Verschieben der Nadelschutzkappe durch den Adapter ist ausgeschlossen.

Erfindungsgemäss umfasst ein Verfahren zur Montage einer Fertigspritze in einer Spritzeneinheit eines Injektionsgeräts mit einem Gehäuse, welches von einem Benutzer greifbar ist und eine Längsachse definiert, folgende Schritte:
- Einsetzen eines Spritzenhalters mit einer starren Halterhülse und einem daran beweglich angebrachten Auflageelement in die Spritzeneinheit des Injektionsgeräts in eine erste axiale Position, in welcher das Auflageelement radial nach aussen auslenkbar ist,
- Blockieren des Spritzenhalters gegen eine Bewegung in distale Richtung,
- Einführen oder Einschieben der Fertigspritze in den Spritzenhalter in distaler Richtung unter radialer Auslenkung des Auflageelements durch eine Nadelschutzkappe der Fertigspritze,
- Freigeben oder Deblockieren einer Bewegung des Spritzenhalters in distale Richtung,
- Bewegen oder Vorschieben des Spritzenhalters und der Fertigspritze in eine zweite axiale Position, in welcher das Auflageelement durch einen Halteabschnitt des Gehäuses gegen ein radiales Auslenken oder Ausscheren in einer Halteposition in Eingriff mit der Spritzenschulter blockiert ist.

Für das Einführen der Fertigspritze in den Spritzenhalter befindet sich der Spritzenhalter ausserhalb des Eingriffs mit dem Halteabschnitt des Gehäuses. Wenn die Fertigspritze vollständig in den Spritzenhalter eingelegt ist und das Auflageelement in die Lücke zwischen der Spritzenschulter und der Nadelschutzkappe eingreift, wird der Spritzenhalter in den Eingriff mit dem Halteabschnitt gebracht. In dieser Eingriffsposition wird verhindert, dass sich das Auflageelement aus dem Eingriff mit der Spritzenschulter quer zur Längsachse bewegt. Ein flexibles Schulterauflageelement zur Ableitung von axialen Kräften eines Produktbehälters wird also auch hier nach Aufnahme von Spritzenhalters und Produktbehälter im Gehäuse durch das Gehäuse selbst gegen laterale Auslenkung gesichert, ohne dass dazu eine manuelle Verschiebung eines separaten Sicherungsrings in distale Richtung notwendig wäre.

In vorteilhaften Weiterentwicklungen des Verfahrens erfolgt das Blockieren des Spritzenhalters mittels eines Montagetools, welches an ein nach distal gerichtetes Abstützelement des Spritzenhalters angreift. Bevorzugt wird das Montagetool seitlich durch ein Sichtfenster im Gehäuse in den Bereich des Spritzenhalters eingeführt und nach erfolgtem Einschieben der Fertigspritze bis in Anschlag mit dem Auflageelement wieder entfernt.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig. 1: eine Explosionsdarstellung eines Autoinjektors,
- Fig. 2: eine Schnittansicht des Autoinjektors aus Fig.1 im Auslieferungszustand,
- Fig.3: einen einteiligen Spritzenhalter,
- Fig.4: einen Montagevorgang einer Fertigspritze im Spritzenhalter aus Fig.3,
- Fig.5: einen zweiteiligen Spritzenhalter mit einem Adapter und einem Adapterhalter,
- Fig.6: eine Schnittansicht des Spritzenhalters aus Fig.5 im Auslieferungszustand,
- Fig.7: eine perspektivische Ansicht von Gehäuses und Endkappe des Autoinjektors aus Fig.1,
- Fig.8: eine Explosionsdarstellung eines Federpakets des Autoinjektors aus Fig.1,
- Fig.9: drei perspektivische Darstellungen des Federpakets aus Fig.8,
- Fig.10: eine perspektivische Darstellung des Federpakets und einer vormontierten Antriebseinheit, und
- Fig.11: eine perspektivische Darstellung eines zweiten Federpakets.

### FIGURENBESCHREIBUNG

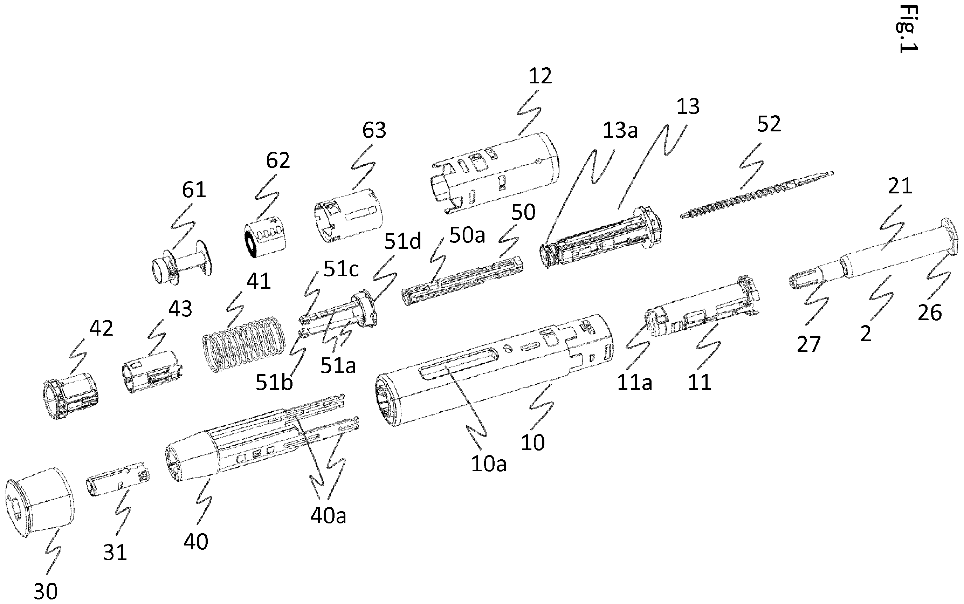

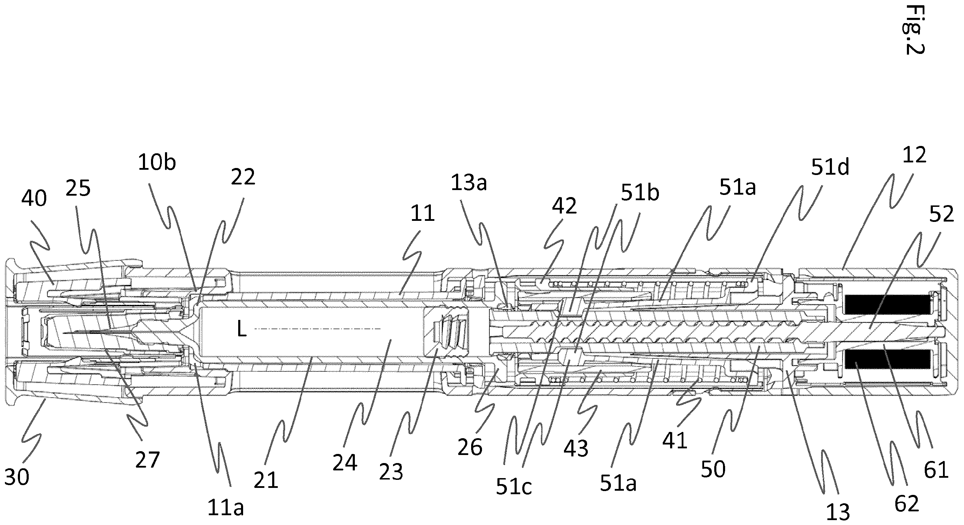

Fig.1 zeigt eine Explosionsdarstellung und Fig.2 eine Längsschnittansicht eines Autoinjektors mit einem hülsenförmigen, zylindrischen Gehäuse 10 mit einer Längsachse L, an welchem ein Spritzenhalter 11, eine Endkappe 12, und ein Mechanikhalter 13 in einem Auslieferungszustand des Autoinjektors fest angeordnet sind. Spritzenhalter 11, Endkappe 12 und Mechanikhalter 13 sind dazu mit dem Gehäuse 10 verrastet, verschnappt, oder anderweitig geeignet dreh- und axialfest montiert.

In dem Spritzenhalter 11 ist eine Fertigspritze 2 aufgenommen und von diesem gehalten. Die Fertigspritze 2 umfasst einen zylindrischen Spritzenkörper 21 als Produktbehälter, in welchem zwischen einer Spritzenschulter 22 und einem entlang der Längsachse L verschiebbaren Stopfen 23 ein Produktaufnahmeraum 24 begrenzt ist. Die Spritzenschulter 22 umfasst einen sich verjüngenden Spritzenabschnitt mit gegenüber dem Spritzenkörper 21 verringertem Querschnitt. An einem distalen Ende des Spritzenkörpers 21 ist eine hohle Injektionsnadel 25 mit der Spritzenschulter 22 fest verbunden, und an einem proximalen Ende der Fertigspritze ist ein Fingerflansch 26 angebracht, welcher radial nach aussen über den Aussenumfang des Spritzenkörpers 21 ragt. Im Produktaufnahmeraum 24 ist im Auslieferungszustand ein abzugebendes Produkt enthalten welches durch Verschiebung des Stopfens 23 in einer Verdrängungsrichtung von einer Stopfenstartposition in eine Stopfenendposition aus dem Produktaufnahmeraum 24 durch die Injektionsnadel 25 heraus verdrängt werden kann.

Die Injektionsnadel 25 der Fertigspritze 2 ist von einer Nadelschutzkappe 27 abgedeckt, welche als sogenanntes Rigid Needle Shield (RNS) ausgebildet ist und ein gummielastisches Nadelschutzelement und eine Hülle aus Hartkunststoff umfasst. Die Nadelschutzkappe 27 schützt die Injektionsnadel 25 gegen mechanische Einwirkungen und Verschmutzung, und hält die Injektionsnadel und das Produkt steril. Zwischen der Spritzenschulter 22 und dem proximalen Ende der Hülle aus Hartkunststoff besteht eine Lücke. An dem distalen Ende des Autoinjektors ist in seinem Auslieferungszustand eine Abziehkappe 30 angeordnet, die vor der Verwendung des Autoinjektors axial abgezogen und/oder abgedreht und vollständig entfernt wird. Die Abziehkappe 30 weist ferner Schnapphaken oder einen separaten, in der Abziehkappe zumindest axial gehaltenen Schutzkappenentferner 31 auf, mit welchem die Nadelschutzkappe 27 von der Fertigspritze 2 gelöst wird.

Die Injektionsnadel 25 ist von einer relativ zum Gehäuse 10 axial verschiebbar gelagerten und in das Gehäuse 10 einschiebbaren Nadelschutzhülse 40 umgeben. In der Ausgangsposition der Nadelschutzhülse 40 steht das distale Ende der Nadelschutzhülse 40 distal über die Nadelspitze der Injektionsnadel 25 über, so dass ein Zugriff auf die Nadelspitze zunächst verhindert wird. Die Nadelschutzhülse 40 weist an ihrer distalen Stirnseite eine Öffnung auf, durch welche die Injektionsnadel 25 bei einer Relativbewegung von Nadelschutzhülse und Injektionsnadel hindurch- und in eine Injektionsstelle eintreten kann. Die Nadelschutzhülse 40 dient auch als Auslöseelement zum Auslösen der Produktausschüttung, wobei die Nadelschutzhülse hierzu unter Spannung einer Nadelschutzhülsenfeder 41 relativ zu dem Gehäuse in die proximale Richtung verschoben wird. Die Nadelschutzhülse umfasst dazu zwei Hülsenarme 40a, welche gegenüber zwei als Sichtfenster bezeichneten Ausnehmungen 10a des Gehäuses um 90° um die Längsachse L versetzt beziehungsweise verdreht angeordnet sind. Nach der erfolgten Injektion kann die Nadelschutzhülse 40 relativ zu dem Gehäuse 10 aus der betätigten Position entlang der Längsachse L in die distale Richtung in eine Nadelschutzposition verschoben und dort gegen erneutes Zurückschieben blockiert werden.

Der Autoinjektor umfasst ein Schaltmodul mit einer Schalthülse 42 und einer von der Schalthülse 42 umgebenen Sperrhülse 43. Die Schalthülse 42 ist mit einem proximalen Ende der Hülsenarme 40a der Nadelschutzhülse und mit einem distalen Ende der Nadelschutzhülsenfeder 41 verbunden. Die Nadelschutzhülsenfeder 41 ist eine als Druckfeder wirkende und als Wendelfeder ausgestaltete Feder aus Metall.

Der Autoinjektor umfasst einen Antrieb mit einer axial verschiebbaren Kolbenstange 50 als Vortriebsglied zur Bewegung des Stopfens 23 in Verdrängungsrichtung. Der Autoinjektor weist ein Halteelement 51 mit zwei flexiblen Haltearmen 51a auf, wobei an einem distalen Ende jedes Haltearms 51a ein erstes Eingriffselement 51b und ein zweites Eingriffselement 51c angeordnet sind. Das erste Eingriffselement 51b weist radial zu der Längsachse L hin und das zweite Eingriffselement 51c weist radial von der Längsachse L weg. Im Auslieferungszustand der Vorrichtung wird das erste Eingriffselement 51b von dem Innenumfang der Sperrhülse 43, der an dem zweiten Eingriffselement 51c anliegt, im Eingriff mit einer Ausnehmung 50a der Kolbenstange gehalten, wodurch eine Bewegung der Kolbenstange 50 relativ zu dem Halteelement 51 in Ausschüttrichtung verhindert wird. Die Nadelschutzhülsenfeder 41 stützt sich mit ihrem proximalen Ende an dem Halteelement 51, insbesondere an einer Abragung 51d, die axial verschiebbar und drehfest in das Gehäuse 10 eingreift, ab.

Der Antrieb umfasst weiter ein Federpaket 6 welches als vorgeladener Energiespeicher in den Autoinjektor montiert wird und als Antriebsmittel für eine Injektion oder Ausschüttung dient. Das Federpaket 6 umfasst einen Federschaft 61, eine Spiral- oder Triebfeder 62, welche im Auslieferzustand mindestens die Energie für eine vollständige Ausschüttung des Produktes im Produktaufnahmeraum 24 speichert, und eine Federhülse 63. Der Antrieb umfasst weiter eine Gewindestange 52 als Rotationsglied, welche mit einem inneren Ende der Spiralfeder 62 gekoppelt ist und mit einem Innengewinde der Kolbenstange 50 in Eingriff steht, so dass eine Drehung der Gewindestange 52 bewirkt, dass die Federenergie an die Kolbenstange 50 übertragen und letztere dadurch in distale Richtung bewegt wird. Die Spiralfeder 62 ist aus einem bandförmigen Material, insbesondere aus Federstahl gewickelt.

Die Fertigspritze 2 ist im Spritzenhalter 11 so aufgenommen, dass sie zumindest gegen eine Bewegung entlang der Längsachse L in distale Richtung relativ zu dem Spritzenhalter 11 gesichert ist. Der Spritzenhalter 11 weist mindestens ein nach innen ragendes und nach proximal gerichtetes axiales Auflageelement 11a auf, an dem sich die Spritzenschulter 22 gegen eine Bewegung in distale Richtung abstützt. Um eine proximale Bewegung der Fertigspritze 2 zu verhindern wird diese durch einen am Fingerflansch 26 angreifenden Haltefederabschnitt 13a des Mechanikhalters 13 in den Eingriff mit dem Auflageelement 11a gedrückt. Durch den Haltefederabschnitt 13a können auch Längenunterschiede des Spritzenkörpers 21, welche aufgrund von Herstellungstoleranzen entstehen, ausgeglichen werden. Zwischen dem Fingerflansch 26 und dem proximalen Ende des Spritzenhalters 11 ist ein Spalt gebildet. Das Gehäuse 10 weist einen ringförmig umlaufenden Halteabschnitt 10b auf, welcher das distale Ende des montierten Spritzenhalters 11 ringförmig umgibt und diesen im Bereich der axialen Auflage 11a gegen radiale Auslenkungen sichert.

Die in Fig.1 und Fig.2 dargestellte Fertigspritze umfasst einen Produktbehälter mit einer vorgegebenen Grösse und einem nominalen Füllvolumen von 2.25 ml, wobei der Spritzenkörper einen gegenüber der Nadelschutzkappe deutlich grösseren Aussendurchmesser aufweist und die Auflageelemente 11a des Spritzenhalters starr ausgebildet sind. Im Folgenden werden zwei Möglichkeiten aufgezeigt, wie in einem Autoinjektor für grossvolumige Fertigspritzen wie vorgehend beschrieben mit minimalsten Anpassungen auch kleinvolumigere Fertigspritzen mit einem Produktbehälter mit einer kleineren Grösse und einem geringeren Aussendurchmesser verwendet werden können.

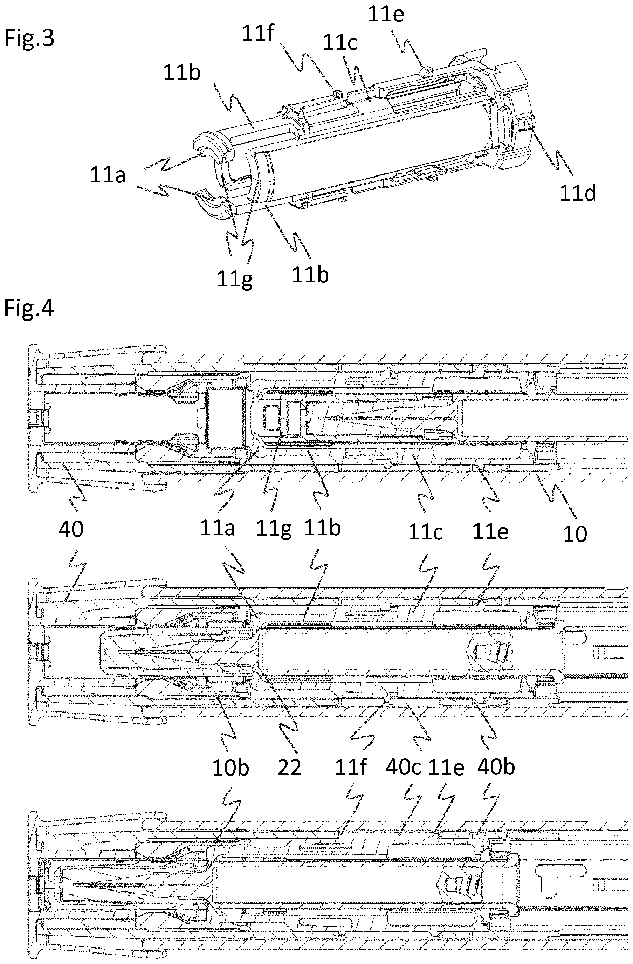

Fig.3 zeigt als erste Variante eine Ausführungsform eines einteiligen Spritzenhalters 11 welche geeignet ist, eine kleinere Fertigspritze in einem Autoinjektor für nominal grössere Produktbehältervolumina zu halten. Der Spritzenhalter umfasst zwei elastische Finger 11b, welche an ihren proximalen Enden an einer Halterhülse 11c des Spritzenhalters befestigt sind und an ihren distalen Enden jeweils ein axiales Auflageelement 11a für eine Spritzenschulter aufweisen. An der Halterhülse 11c sind weiter angebracht zwei einander gegenüberliegende erste Schnappelemente 11d zum Eingriff in das Gehäuse 10 sowie jeweils zwei einander gegenüberliegende zweite und dritte Schnappelemente 11e, 11f zum Eingriff in Ausnehmungen der Nadelschutzhülse 40. Eigenschaften der Auflageelemente 11a und Funktionen der Schnappelemente welche für mehrere Varianten zutreffen sind im Anschluss an die Beschreibung der Varianten zusammengefasst.

Fig.4 zeigt drei Schritte der erfindungsgemässen Montage des Spritzenhalters aus Fig.3. Als erstes wird der Spritzenhalter 11 in das Gehäuse 10 axial eingeführt und in einer ersten axialen Position gehalten (Fig.4 oben). In dieser Position sind die distalen Enden der Finger 11b mit den Auflageelementen 11a in der Lage radial nach aussen ausgelenkt zu werden, unter Bildung einer ausreichenden Öffnung für die Passage der Nadelschutzkappe 27. Insbesondere werden die Finger 11b nicht durch einen Halteabschnitt des Gehäuses an dieser Bewegung gehindert. Der Spritzenhalter wird in der ersten Position vorläufig gehalten durch eine leicht lösbare Schnappverbindung zwischen Spritzenhalter und Gehäuse, umfassend die beiden zweiten, radial nach aussen weisenden Schnappelemente 11e des Spritzenhalters und dazu komplementäre erste Ausnehmungen 40b der Nadelschutzhülse. Als Folge der abgeschrägten Form der distalen Halteflächen der Schnappelemente 11e ist diese Halterung nicht ausreichend für eine Aufnahme der Kräfte welche beim Einpressen der Fertigspritze auf den Spritzenhalter wirken, so dass für diesen Schritt ein Montagewerkzeug von aussen in das Gehäuse 10 eingeführt und mit Abstützelementen 11g des Spritzenhalters 11 in Eingriff gebracht wird. Die Abstützelemente 11g können durch geeignete, nach distal gerichtete starre Flächen, Kanten, Auskragungen, oder sonstige Vorsprünge an der Halterhülse 11c gebildet werden. Vorliegend sind dazu zwei Kanten am distalen Ende der Halterhülse, zwischen den Fingern 11b beziehungsweise gegenüber den Fingern um 90° um die Längsachse L rotiert, vorgesehen. Diese Kanten sind gegenüber den Auflageelementen 11a in proximaler Richtung soweit versetzt, dass sich in der ersten Position zwischen den Kanten und dem distalen Rahmen der Ausnehmungen 10a der Sichtfenster je eine Öffnung bildet. Durch diese Öffnungen kann ein zweiteiliges Montagewerkzeug beidseits von ausserhalb und rechtwinklig zur Längsachse L in gerader Linie bis zum Eingriff mit den Abstützelementen 11g eingeführt werden. Die axiale Position des Montagewerkzeugs ist in Fig.4 (oben) schematisch mittels strichpunktierter Linien angedeutet, es ragt dabei keinesfalls radial bis in den Innenraum des Spritzenhalters.

Als zweiter Schritt wird die Fertigspritze axial in den Spritzenhalter eingesetzt (Fig.4 Mitte) wobei die Auflageelemente 11a erst zur Seite gedrängt werden und anschliessend hinter der Nadelschutzkappe 27 in die Lücke zwischen Nadelschutzkappe und Spritzenschulter 22 greifen. Anschliessend wird das Montagewerkzeug wieder entfernt. Schlussendlich werden Spritzenhalter und Fertigspritze zusammen nach distal in eine zweite axiale Position geschoben (Fig.4 unten). In dieser sind die flexiblen Finger 11b mit den Auflageelementen 11a von dem Halteabschnitt 10b des Gehäuses mit einem im Vergleich zur ersten Position geringeren Innendurchmesser gehalten und gegen ein radiales Auslenken der Ausscheren blockiert. Der Spritzenhalter ist in der zweiten Position mit den beiden ersten Schnappelementen 11d in dazu komplementären Ausnehmungen des Gehäuses 10 gegen weitere axiale Bewegungen unlösbar verschnappt. Durch das Schieben von Spritzenhalter und Fertigspritze zusammen nach distal in die zweite axiale Position wird der Schutzkappenentferner 31 in distaler Richtung spielfrei an zumindest einen Anschlag an der Abziehkappe 30 gedrängt.

An Stelle der zwei einander gegenüberliegenden Abstützelemente 11g können auch ein einziges Abstützelement oder mehr als zwei Abstützelemente genutzt werden. Der Abstützvorgang ist auf jeden Fall bevorzugt angepasst an geeignete Ausnehmungen im Gehäuse und/oder an Fähigkeiten des Montagewerkzeugs, mit dem Ziel eines einfach zu realisierenden Montage-Zwischenschritts. Falls das Montagewerkzeug vor der Einführung des Spritzenhalters platziert wird kann auch auf die zweiten Schnappelemente 11e zur Vorpositionierung des Spritzenhalters verzichtet werden.

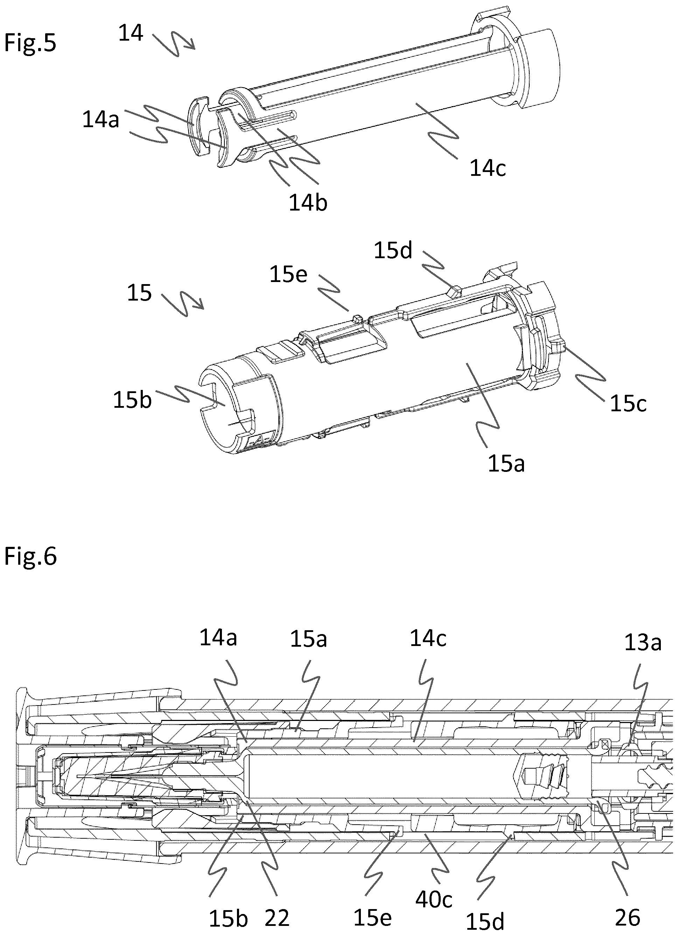

Fig.5 und Fig.6 zeigen als zweite Variante eine zweiteilige oder modulare Ausführungsform mit einem Adapter 14 und einem Adapterhalter 15 welche geeignet ist, eine kleinere Fertigspritze in einem Autoinjektor für nominal grössere Produktbehältervolumina zu halten. Der Adapter 14 (Fig.5 oben) umfasst einen Adapterkörper 14c mit einem an die aufzunehmende kleinvolumige Fertigspritze angepassten Innendurchmesser und einen Aussendurchmesser, welcher wiederum einem Innendurchmesser einer Halterhülse 15a des Adapterhalters 15 (Fig.5 unten) entspricht. Im montierten Zustand (Fig.6) nimmt der Adapterkörper 14c den Spritzenkörper 21 der Fertigspritze auf, während die Halterhülse 15a den Adapterkörper 14c radial umfasst und im Gehäuse 10 des Autoinjektors gehalten ist. Der Adapterhalter 15 ist von einem Spritzenhalter des Injektionsgeräts für eine grossvolumige Fertigspritze verschieden, entsprechend kann der Innendurchmesser des Adapterhalters grösser oder kleiner als ein Aussendurchmesser der grossvolumigen Fertigspritze gewählt werden.

Der Adapterkörper 14c weist zwei Adapterarme auf, welche an ihrem distalen Ende über Stege und an ihrem proximalen Ende über einen Ansatz verbunden sind. Die Arme definieren und begrenzen in Richtung der Längsachse L zwei longitudinale Ausnehmungen im Adapterkörper 14c welche im montierten Zustand je mit einem der Sichtfenster 10a des Gehäuses ausgerichtet sind dadurch den Blick auf den Spritzenkörper 21 der Fertigspritze nicht behindern. Der proximale Ansatz bildet eine Adapterschulter und einen Innenraum mit einem gegenüber dem Adapterkörper vergrösserten Durchmesser zur Aufnahme des Fingerflansches 26 der Fertigspritze. Der Adapter 14 umfasst weiter zwei elastische Finger 14b, welche an ihren proximalen Enden am Adapterkörper 14c befestigt sind und an ihren distalen Enden jeweils ein axiales Auflageelement 14a für eine Spritzenschulter aufweisen.

Die Halterhülse 15a umfasst einen ringförmig umlaufenden Halteabschnitt 15b an ihrer Innenseite, welcher das distale Ende des montierten Adapters 14 ringförmig umgibt und die Auflageelemente 14a gegen radiale Auslenkungen sichert. Das distale Ende der Halterhülse 15a weist selbst keine Halteelemente oder Verjüngungen auf. Dadurch steht der Adapter 14 mit den distalen Enden der Auflageelemente 14a unmittelbar selbst am Gehäuse 10 des Autoinjektors an, und axiale Kräfte auf den Produktbehälter der Fertigspritze 2 werden von der Spritzenschulter 22 über die Auflageelemente 14a zum Gehäuse abgeleitet. Der Fingerflansch 26 berührt in dieser Position die Adapterschulter nicht und wird weder durch den Adapteransatz noch durch den Adapterhalter oder sonst ein Gehäuseteil an einer Rotation gehindert, so dass sich die montierte Fertigspritze frei drehen kann. An der Halterhülse 15a sind weiter angebracht zwei einander gegenüberliegende erste Schnappelemente 15c zum Eingriff in das Gehäuse 10 sowie jeweils zwei einander gegenüberliegende zweite und dritte Schnappelemente 15d, 15e zum Eingriff in Ausnehmungen der Nadelschutzhülse 40.

Zur Montage der Fertigspritze wird zuerst der Adapterhalter 15 in das Gehäuse 10 des Autoinjektors eingeführt und mit zwei einander gegenüberliegenden ersten Schnappelementen 15c in einer dazu komplementären Ausnehmung des Gehäuses unlösbar axial und rotationsfest verschnappt. Anschliessend wird ausserhalb des Injektionsgeräts die Fertigspritze 2 axial in distaler Richtung in den Adapter 14 eingeführt. Dabei wird die Nadelschutzkappe 27 durch eine durch die Finger 14b gebildete Öffnung hindurchgepresst wobei die Finger 14b durch die Hülle der Nadelschutzkappe radial nach aussen ausgelenkt werden. Sobald das proximale Ende der Nadelschutzkappe die Öffnung passiert hat schnappen die Finger radial nach innen und platzieren die Auflageelemente 14a in einer Halteposition in Eingriff mit der Spritzenschulter 22. Zuletzt werden Adapter und Spritze in einer rotativ korrekten Position in den Adapterhalter eingeführt. Um eine proximale Bewegung der Fertigspritze 2 zu verhindern wird diese durch einen am Fingerflansch 26 angreifenden Haltefederabschnitt 13a des Mechanikhalters 13 in den Eingriff mit dem Auflageelement 14a gedrückt.

In beiden vorgängig genannten Varianten umfasst jedes Auflageelement 11a, 14a einen Abschnitt oder einen Sektor eines Kreisringes mit einem mittleren Durchmesser entsprechend einem Durchmesser der Spritzenschulter. In Rotationsrichtung um die Längsachse L ist die Ausdehnung des Auflageelements und/oder eine mittlere Bogenlänge des Abschnitts grösser als die Breite des Fingers, das Auflageelement steht in beide Rotationsrichtungen vom Finger ab so dass Finger und Auflageelement zusammen die Form eines Ankers aufweisen. Bei der Montage einer Fertigspritze wird deren Nadelschutzkappe durch die durch die Finger 11b, 14b gebildete Öffnung hindurchgepresst wobei die Finger durch die Hülle der Nadelschutzkappe radial nach aussen ausgelenkt werden. Sobald das proximale Ende der Nadelschutzkappe die Öffnung passiert hat schnappen die Finger radial nach innen und platzieren die Auflageelemente 11a, 14a in einer Halteposition in Eingriff mit der Spritzenschulter. Dadurch kann die durch die Innenkanten der Auflageelemente 11a, 14a gebildete Öffnung im Eingriff einen Durchmesser aufweisen, welcher kleiner ist als ein Aussendurchmesser der Nadelschutzkappe. Die Auflageflächen der Auflageelemente 11a, 14a kommen somit zumindest teilweise in die Lücke zwischen Nadelschutzkappe und Spritzenschulter zu liegen, und ermöglichen auch eine Aufnahme von axialen Kräften der Fertigspritze falls deren Aussendurchmesser nur unwesentlich oder gar nicht grösser ist als der Durchmesser der Nadelschutzkappe. Durch einen Eingriff sichernden Halteabschnitt 10b, 15b des Gehäuses und des Adapterhalters, welcher an einem Aussenumfang der Finger 11b, 14b unter wenig Spiel anliegt, werden die Finger daran gehindert, sich quer zur Längsachse zu bewegen und dabei die axiale Auflage der Spritzenschulter zu kompromittieren.

Alternativ zu den dargestellten zwei Auflageelementen 11a, 14a auf zwei unterschiedlichen Fingern 11b, 14b kann auch nur ein einziges Auflageelement in Form eines radial nach innen gerichteten Kragens oder einer Verjüngung vorgesehen sein, welcher durch die Nadelschutzkappe temporär ausgeweitet werden kann. Der Kragen ist dazu durch einen Schlitz in radialer Richtung unterbrochen und bildet einen C-förmigen, mehr als 180° überdeckenden Kreisbogenabschnitt welcher ebenfalls senkrecht zur Längsachse L durch die Nadelschutzkappe elastisch gespreizt und weggedrängt werden kann. Es können auch mehr als zwei Finger vorgesehen sein, und/oder an Stelle von ankerförmigen Auflageelementen auch nur Auflageelemente mit der Breite des jeweiligen Fingers. Das Raumvolumen in welches der oder die Finger 11b der ersten Variante beim Einführen der Fertigspritze ausweichen, und der Halteabschnitt 10b, 15b des Gehäuses und des Adapterhalters können entsprechend der Anzahl Finger und der Ausdehnung der Auflageelemente unterteilt und/oder in der Form angepasst werden. An Stelle der elastischen Finger sind auch flexible, nicht-elastische Finger denkbar, beispielsweise mit einem Gelenk, vorzugsweise einem Filmgelenk, beim Übergang zur Halterhülse oder zum Adapterkörper. In diesem Fall wird das Auflageelement erst durch geeignete Abschrägungen am proximalen Ende des Halteabschnitts des Gehäuses oder des Adapterhalters bei der finalen Bewegung der Fertigspritze in den Eingriff mit der Spritzenschulter geführt.

Durch die Verwendung eines Spritzenhalters gemäss der ersten Variante oder eines modularen Spritzenhalters gemäss der zweiten Variante kann ein Injektionsgerät, welches für nominal grossvolumige Produktbehälter konzipiert wurde, auch mit kleinvolumigen Produktbehältern eingesetzt werden. Der Übergang zu einem Produktbehälter mit einem kleineren Durchmesser und einer kürzeren Länge erfordert dabei zusätzlich zumindest oder maximal einen Austausch der Kolbenstange und des Mechanikhalters. Eine Kolbenstange mit einem kleineren Durchmesser passt in den schmaleren Produktbehälter, und ein längerer Mechanikhalter und/oder ein längerer Haltefederabschnitt vermag auch einen kürzeren Produktbehälter mit ausreichend Kraft nach distal in Eingriff mit dem Auflageelement zu drängen.

Der Spritzenhalter 11 der ersten Variante und der Adapterhalter 15 der zweiten Variante sind für nachfolgende Zusatzfunktionen im montierten Zustand vorgesehen. Die zweiten Schnappelemente 11e, 15d des Spritzenhalters und des Adapterhalters greifen in dazu komplementäre zweite, schlitzförmige Ausnehmungen 40c der Nadelschutzhülse ein. In der Ausgangsposition der Nadelschutzhülse 40 (Fig.4 unten, Fig.6) und/oder in der Nadelschutzposition der Nadelschutzhülse liegen die proximalen Enden der schlitzförmigen Ausnehmungen 40c an proximalen Stoppflächen der zweiten Schnappelemente 11e, 15d an, wodurch eine Bewegung der Nadelschutzhülse in die distale Richtung verhindert wird. Die dritten Schnappelemente 11f, 15e des Spritzenhalters und des Adapterhalters in Gestalt von federnd an der Halterhülse 11c, 15a angeordneten Nocken greifen ebenfalls in die zweiten Ausnehmungen 40c ein, alternativ in eine andere schlitzförmige Ausnehmung der Nadelschutzhülse. Die Nocken sind so geformt, dass bei dem Versuch, die Nadelschutzhülse 40 aus der Ausgangsposition in die betätigte Position zu verschieben, die Nocken die Verschiebung der Nadelschutzhülse zunächst verhindern. Sobald die zum Zurückschieben aufgebrachte Kraft einen gewissen Schwellwert überschreitet werden die Nocken durch die distalen Enden der Ausnehmung 40c radial nach innen aus dem Eingriff herausgedrückt, wodurch die Nadelschutzhülse 40 schlagartig in die betätigte Position geschoben und die Injektionsnadel 25 in die Einstichstelle eingestochen wird.

Die ersten, zweiten, und dritten Schnappelemente 11d, e, f und 15c, d, e sind jeweils paarweise vorgesehen auf einander gegenüberliegenden Seiten des Spritzenhalters und des Adapterhalters, wobei auch nur je ein Schnappelement oder mehr als zwei über einen Umfang verteilte Schnappelemente möglich sind. Der Spritzenhalter 11 und der Adapterhalter 15 sind bevorzugt aus einem Kunststoff gefertigt mit im Vergleich zum Gehäuse 10 unterschiedlichen Materialeigenschaften, insbesondere im Hinblick auf die Ausbildung der elastischen Schnappelemente. Der Spritzenhalter 11 und der Adapterhalter 15 können im Gegensatz zum Gehäuse auch optisch transparent sein und dadurch als Berstschutz für den Produktbehälter wirken ohne die Sicht durch die Ausnehmungen der Sichtfenster 10a im Gehäuse auf das Produkt zu beeinträchtigen.

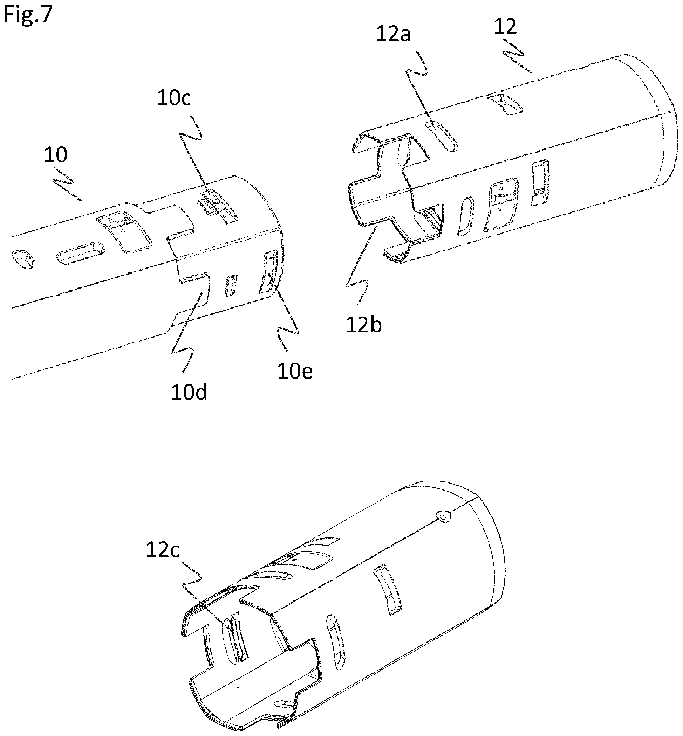

Fig.7 zeigt eine perspektivische Ansicht des Gehäuses 10 und der Endkappe 12 in axial getrennter Lage und in für die Endmontage korrekter Drehposition (oben). Im Auslieferungszustand sind alsdann Gehäuse und Endkappe formschlüssig dreh- und axialfest miteinander verbunden so dass die Endkappe das Gehäuse verschliesst. Das Gehäuse 10 weist dazu Schnappelemente 10c auf, welche in Öffnungen oder Ausnehmungen 12a der Endkappe 12 eingreifen und zumindest für eine axiale Sicherung sorgen. Das distale Ende der Endkappe weist in Richtung der Längsachse L eine nicht-rotationssymmetrische Kappenkontur 12b auf welche auf eine komplementäre Gehäusekontur 10d des Gehäuses abgestimmt ist und dadurch für eine rotativ korrekte Ausrichtung der Endkappe bei der Zusammenführung von Gehäuse und Endkappe sorgt, und im verschnappten Zustand zur Drehsicherung beiträgt. Das Gehäuse weist eine über die Gehäusekontur in proximaler Richtung hinausgehende Verlängerung oder Erweiterung auf welche im verschnappten Zustand als Ganzes von der Endkappe umfasst wird. Die Verlängerung dient der radialen Zentrierung der Endkappe bei der Zusammenführung von Gehäuse und Endkappe und trägt die Schnappelemente 10c. Die Verlängerung weist einen Querschnitt auf welcher wie vorliegend insbesondere dieselbe Querschnittsform aufweist wie das Gehäuse, und zumindest auf einen Innenquerschnitt der Endkappe abgestimmt ist, und somit ebenfalls zur Drehsicherung beitragen kann.

Im gezeigten Fall weisen Gehäuse, Verlängerung, und Endkappe einen quadratischen Querschnitt auf mit leicht konvex abgerundeten Seiten und einer auf vier 90° Schritte beschränkten diskreten Rotationssymmetrie. Demgegenüber weisen die Konturen 10d, 12b nur eine 180° Symmetrie auf, da nur jeweils die Konturabschnitte auf zwei gegenüberliegenden Seiten identisch sind. Entsprechend passen in einer Vormontageposition, in welcher die Endkappe gegenüber der Drehposition für die Endmontage um 90° gedreht ist (Fig.7 unten), die Vorsprünge der Gehäusekontur 10d nicht in die Aussparungen der Kappenkontur 12b, so dass die Endkappe nur bis zu einer Zwischenposition auf die Gehäuseverlängerung aufgeschoben werden kann. In dieser Vormontageposition schnappt ein Haltenocken 12c auf der Innenseite der Endkappe in eine Ausnehmung 10e der Gehäuseverlängerung, so dass die Endkappe zumindest für Transportzwecke ausreichend fest am Gehäuse gehalten ist, aber für die Endmontage auch leicht wieder aus dieser Verbindung gelöst werden kann.

Die beschriebenen Zentrier-, Ausrichtungs-, und Schnappelemente können auch an der jeweils anderen Komponente angebracht sein, beispielsweise ist eine konzentrische distale Verlängerung der Endkappe denkbar, und/oder die Schnappelemente sind an der Endkappe angebracht und greifen in eine Öffnung des Gehäuses ein. Alternativ zur einer über den Gehäuseumfang kontinuierlichen Verlängerung sind auch axial gerichtete Arme denkbar, welche vom proximalen Ende des Gehäuses oder vom distalen Ende der Endkappe abstehen, die Schnappelemente tragen und für eine Zentrierung und/oder Ausrichtung der Endkappe bei der Zusammenführung sorgen. An Stelle der lösbaren Haltenocken können andere Verbindungsmittel wie Rastelemente oder Bajonett- und Gewindeverbindungen für die Vormontage vorgesehen sein, und an Stelle der Schnappelemente können auch nicht lösbare Verbindungsmittel verwendet werden.

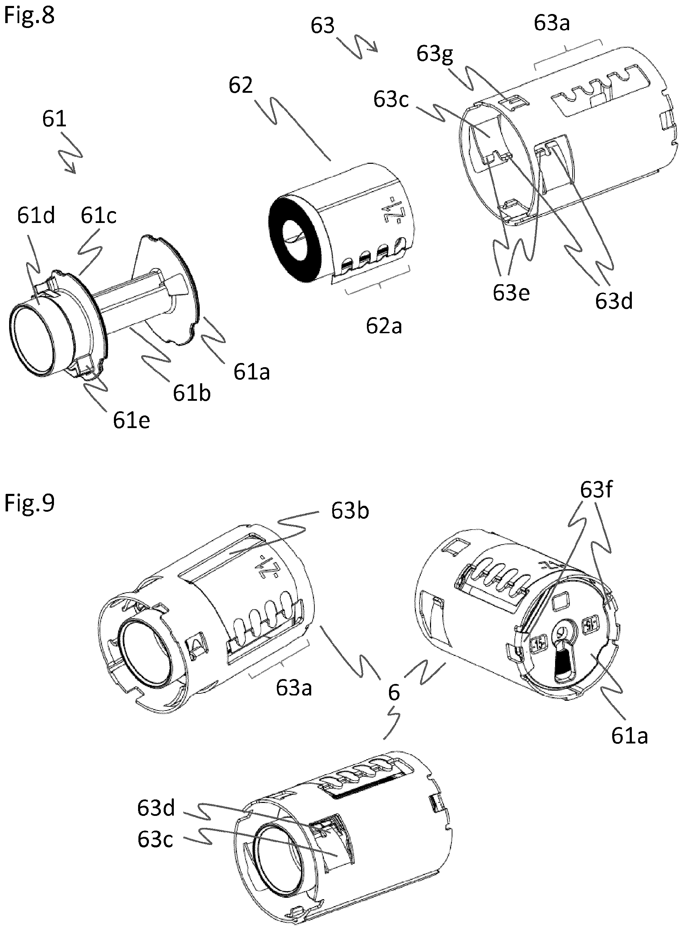

Fig.8 zeigt eine Explosionsdarstellung des Federpakets 6 des Autoinjektors aus Fig.1. Das Federpaket umfasst einen Federschaft 61, eine Spiralfeder 62, und eine Federhülse 63. Der einstückige Federschaft 61 umfasst einen proximalen Endflansch 61a, eine Federachse 61b, einen distalen Endflansch 61c, und eine hülsenförmige distale Verlängerung 61d als Führung und Positionierung zu einem Mechanikhalter. Am distalen Endflansch befindet sich ein Anschlag 61e mit einer radial ausgerichteten Anschlagsfläche. Der Federschaft 61 weist eine Bohrung in Richtung der Federachse auf in welcher bei Montage des Federpakets 6 auf eine Antriebseinheit ein proximales Ende einer Gewindestange drehfest aufgenommen werden kann. Die Spiralfeder 62 ist aus einem Federband aus Federstahl spiralförmig gewickelt und mit einem inneren Ende in der Federachse 61b drehfest verankert, führt im ungespannten Zustand in zwölf Windungen um die Federachse, und ist mit einem äusseren Ende mit der Federhülse 63 verbunden. Der Abstand zwischen den zwei Endflanschen 61a, 61c entspricht der Länge der Federachse 61b und der Breite des Federbandes. Das innere Ende der Wicklung umfasst nicht dargestellte Ausnehmungen oder Haltelaschen im Federband zum Einhaken oder Einführen in entsprechende Gegenelemente der Federachse 61b, so dass zumindest Torsionskräfte oder ein Drehmoment zwischen Federschaft und Spiralfeder übertragen werden können. Die Federhülse 63 ist aus einem Hülsenmantel aus Blech als Kreiszylinder geformt, mit einer Zylinderhöhe entsprechend einer Länge des Federschaftes 61. Die Federhülse umfasst zwei einander gegenüberliegende Sperrschnapper 63c mit jeweils einem flexiblen Arm und einem nach innen weisenden Zahn 63d am freien Ende des Arms. Neben dem Zahn 63d und als Teil desselben flexiblen Arms des Sperrschnappers ist ein Steuerelement 63e in Form einer nach aussen abgewinkelten Lasche vorhanden. Die Federhülse umfasst auch zwei einander gegenüberliegende und nach innen weisende Stoppelemente 63g.

Das Federband wird als ebenes Metallband in der Federachse 61b verankert und dann zwischen den zwei dauerhaft fest mit der Federachse verbundenen Endflanschen 61a, c um den Schaft gewickelt. Anschliessend werden Federschaft 61 und Spiralfeder 62 in proximale Richtung in die Federhülse 63 eingeführt und die Spiralfeder mit der Federhülse verbunden. Dazu weist das äussere Ende der Spiralfeder 62 vier in Richtung der Federachse neben einander liegende Öffnungen, Löcher oder Ösen 62a im Federband auf, und die Federhülse 63 umfasst vier in Richtung der Federachse neben einander in Form eines Kamms angeordnete Zacken oder Zähne 63a. Die vier Zacken 62a weisen in Federspannrichtung und sind gebildet durch eine komplementäre Ausnehmung im Hülsenmantel. Die Öffnungen 62a der Spiralfeder werden unter leichter Spannung der Spiralfeder über die Zacken 63a der Federhülse gezogen und in die Zacken eingehängt oder mit diesen verhakt. Durch den Eingriff der Verzahnung erfolgt eine axiale Positionierung des Federbandes mit der Federhülse.

Fig.9 links zeigt eine erste perspektivische Darstellung des Federpakets aus Fig.8. Das äussere Ende der Spiralfeder 62 ist mittels der vier Öffnungen 62a im Federband in die vier Zacken 63a der Federhülse eingehängt. Das Federband führt dazu zuerst durch einen von der Ausnehmung verschiedenen Schlitz 63b der Federhülse nach aussen, so dass der Steg, welcher die Zacken zwischen Schlitz und Ausnehmung untereinander verbindet, in Fig.9 vom Federband überdeckt ist. Das Federband weist im Bereich der Öffnungen 62a eine in Federspann- oder Wicklungsrichtung nach innen führende Treppenstufe mit einer radialen Stufenhöhe entsprechend der Dicke von Federband und Hülsenmantel auf, so dass das Federband beidseits der Stufe praktisch ausschliesslich tangential zum Hülsenmantel ausgerichtet ist. Die Treppenstufe umfasst zwei Biegekanten im Federband, dazwischen ist ein Stufenabsatz des Federbands gegenüber der Federspannrichtung um nicht mehr als 90° abgewinkelt. Dieser Stufenabsatz liegt zwischen den Zacken am Steg an.

Fig.9 rechts zeigt eine zweite perspektivische Darstellung des Federpakets aus Fig.8 aus einem anderen Blickwinkel. Deutlich zu sehen ist der proximale Endflansch 61a. Dieser und damit auch der Federschaft wird durch radial nach innen weisende Haltestrukturen 63f der Federhülse gegen proximale Bewegungen gehalten. Der proximale Endflansch weist einen radial verlaufenden Spalt oder ein Sichtfenster zur optischen Kontrolle der Spiralfeder auf.

Alternativ zu den im Hülsenmantel ausgebildeten Zacken können radial vom Hülsenmantel abstehende Haken an diesem angebracht oder angeformt sein. Werden die Haken auf der Innenseite der Federhülse angebracht sind keine Ausnehmungen oder Schlitze im Hülsenmantel erforderlich. Das Federband kann auch durch die Ausnehmung nach aussen geführt werden, bevorzugt mittels einer in Federspannrichtung nach aussen führenden Treppenstufe, so dass auf den separaten Schlitz verzichtet werden kann. Ebenso können auch mehr oder weniger als vier Öffnungen und Zacken vorgesehen sein, und/oder andere Formen von Öffnungen als kreisrund, insbesondere solche mit einer Kante parallel zur Federachse, zusammen mit entsprechend breiter ausgebildeten Zacken im Hülsenmantel. Die Anzahl der Öffnungen kann die Anzahl der Zacken auch übertreffen. Öffnungen, Ausnehmung und Steg können durch Ausstanzen oder Laserschneiden aus dem Federband der Spiralfeder und dem Blech des Hülsenmantels einfach gefertigt werden.

Fig.9 unten zeigt eine dritte perspektivische Darstellung des Federpakets aus Fig.8 in einer gegenüber der ersten Darstellung um 90° um die Federachse gedrehten Position. Die Spiralfeder 62 kann durch Rotation der Federhülse 63 im Gegenuhrzeigersinn (distale Blickrichtung) relativ zum Federschaft 61 gespannt werden und im Endzustand beispielsweise dreimal mehr Windungen bilden als im ungespannten Zustand. Die resultierende Vorspannung entspricht einem Drehmoment von 1 bis 100 Nmm, bevorzugt einem Drehmoment von 30 bis 80 Nmm, und besonders bevorzugt einem Drehmoment von 60 bis 70 Nmm. Durch geeignete Massnahmen kann im Federpaket die beim Spannvorgang erzeugte potentielle Energie gespeichert und das Federpaket bei gespannter Spiralfeder auch als Schüttgut transportiert oder aufbewahrt werden. Dazu umfassen der distale Endflansch 61c und das distale Ende der Federhülse 63 Sicherungselemente zur Sicherung einer einmal aufgeladenen Federspannung, und/oder Kopplungselemente zur lösbaren Kopplung von Federflansch und Federhülse. Die Sicherungselemente umfassen den Anschlag 61e mit einer radial ausgerichteten Anschlagsfläche am distalen Endflansch, in welche der nach innen weisenden Zahn 63d am freien Ende des flexiblen Arms des Sperrschnapper 63c der Federhülse eingreifen kann. Die Sperrrichtung des Eingriffs von Anschlag 61e und Zahn 63d ist dabei so gewählt, dass eine Entspannung der Spiralfeder 62 verhindert wird, und die Federhülse 63 relativ zum Federschaft 61 gegen eine Rotation oder Verdrehung durch ein Drehmoment der Spiralfeder gesichert ist. In dieser Position begrenzt der Arm des Sperrschnappers auch eine Bewegung des distalen Endflansches 61c in distale Richtung, analog zur proximal begrenzenden Haltestruktur 63b der Federhülse. Eine Bewegung des Steuerelements 63e in eine radiale Freistellungsrichtung hat eine Bewegung des Zahnes 63d in dieselbe Richtung zur Folge, durch eine radiale Freistellungsbewegung des Steuerelements kann also die Verdrehsicherung gelöst werden.

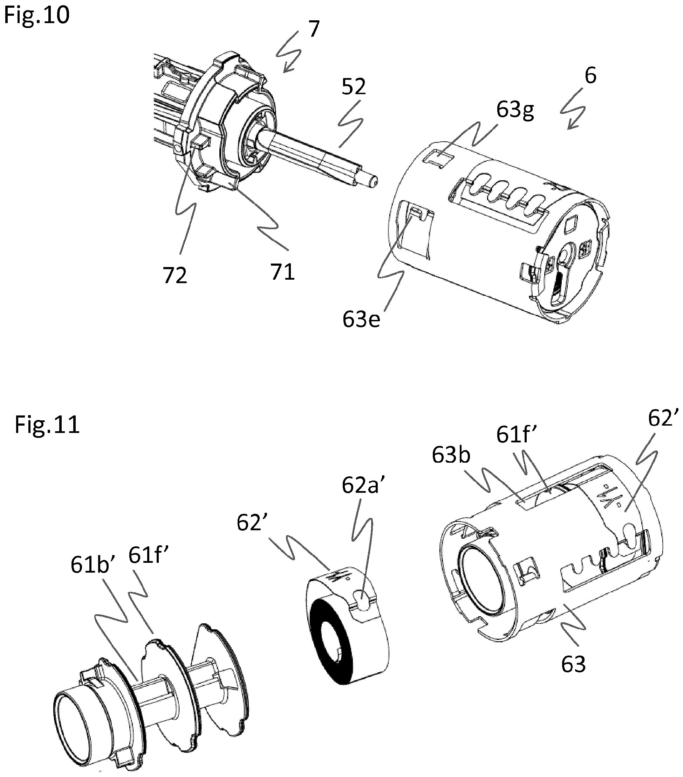

Fig.10 zeigt eine perspektivische Darstellung des Federpakets 6 und einer vormontierten Antriebseinheit 7 des Injektionsgerätes in einem getrennten Zustand. Bei der Montage des Federpakets auf die Antriebseinheit wird die Gewindestange 52 in die Bohrung des Federschafts 61 eingeführt und ein Freigabeelement 71 der Antriebseinheit radial zwischen Anschlag 61e und Federhülse 63 und um die Federachse 61b leicht versetzt zum Steuerelement 63e positioniert. Bei einer anschliessenden Verdrehung des Federpakets 6 relativ zur Antriebseinheit 7 greift das Freigabeelement 71 in das Steuerelement 63e und bewegt dieses, und damit auch den Zahn 63d, radial in Freistellungsrichtung. Dadurch wird die Federhülse zur Rotation gegenüber dem durch die Antriebseinheit drehfest aufgenommenen Federschaft freigegeben, durch eine leichte Entspannung der Spiralfeder wird die Federhülse rotiert, bis ein nach innen weisendes Stoppelement 63g der Federhülse an einem Gegenanschlag 72 der Antriebseinheit anschlägt und eine kraft- und drehmomentschlüssige Verbindung zwischen Federpaket und Antriebseinheit erzeugt wird. Ab diesem Moment ist das im Federpaket gespeicherte Drehmoment an die Antriebseinheit gekoppelt und wird durch Sperrelemente in der Antriebseinheit bis zur Auslösung einer Produktausschüttung gesichert.

In einer bevorzugten Variante ist die Federhülse 63 aus einem metallischen Blech geformt, aus welchem der Sperrschnapper 63c als flexibler Arm zusammen mit Zahn und Steuerelement durch Ausstanzen oder Laserschneiden ausgeformt wird. Am freien Ende des Armes werden anschliessend in Art einer Lasche der Zahn 63d nach innen und das Steuerelement 63e nach aussen abgebogen. Auch das Stoppelement 63g der Federhülse ist aus dem Hülsenmantel ausgeschnitten und nach innen abgebogen. In den gezeigten Ausführungsformen sind über den Umfang des Federpakets verteilt und untereinander jeweils um 180° versetzt zwei Sperrschnapper und zwei Stoppelemente mit entsprechenden Anschlägen vorgesehen. Dabei kann auch nur ein Sperrschnapper und/oder ein Stoppelement vorgesehen sein, oder mehr als zwei Sperrschnapper und/oder Stoppelemente.

Nach erfolgter maximaler Spannung der Spiralfeder wird der Zahn 63d beispielsweise durch eine plastische Deformation des Armes des Sperrschnappers 63c mit dem Anschlag 61e in Eingriff gebracht. Alternativ ist auch eine elastische Deformation des Armes denkbar, wobei der Sperrschnapper durch das Steuerelement 63e im Eingriff mit dem Anschlag blockiert wird. Das Steuerelement 63e weist zumindest eine geeignete Eingriffsfläche für das Freigabeelement 71 in Form einer Gewindefläche, Abschrägung oder eines Keils auf. Die sicherungslösende Auslenkung des Steuerelements kann in radialer Richtung wie gezeigt, oder auch in axialer oder tangentialer Richtung erfolgen. Die Sicherungselemente zur Verdrehsicherung können auch einen flexiblen Sperrschnapper am Schaft und einen Anschlag an der Federhülse umfassen. Anschlag oder Sperrschnapper können auch unmittelbar am Federschaft, an einem mit dem Schaft drehfest verbundenen weiteren Flansch, oder an einer mit dem Schaft drehfest verbundenen Speiche, in einem beliebigen radialen Abstand vom Schaft platziert sein. Der komplementäre Sperrschnapper oder Anschlag kann in entsprechendem Abstand von der Federachse an einem mit der Federhülse drehfest verbundenen Drehmomentübertragungsmittel angebracht sein. Ebenso können das Stoppelement der Federhülse und der komplementäre Gegenanschlag der Antriebseinheit auch in geringerem Abstand zur Federachse angeordnet sein. Das Freigabeelement kann das Steuerelement bei der Verdrehung des Federpakets anstatt radial auch axial auslenken oder wegschieben. Die Freigabebewegung des Steuerelements kann auch bereits während der axialen Bewegung von Federpaket und Antriebseinheit erfolgen, so dass die anschliessende Verdrehung unterbleiben kann. Zumindest der proximale Endflansch 61a als radiale Begrenzungsfläche könnte auch mit der Federhülse an Stelle des Federschafts fest verbunden sein.

Fig.11 zeigt eine perspektivische Darstellung eines zweiten Federpakets mit einer zweiten Spiralfeder 62' aus einem Federband mit einer gegenüber dem Federpaket aus Fig.8 geringeren Breite. Ein zwischen den Endflanschen an der Federachse 61b' angebrachter Zwischenflansch 61f' begrenzt zusammen mit dem proximalen Endflansch 61a' das Federvolumen. Die Federhülse 63 ist identisch mit jener des Federpakets aus Fig.8. Das Federband weist nur eine statt vier Öffnungen 62a' auf, deutlich sichtbar ist dadurch die distale Hälfte des zackenverbindenden Stegs im Hülsenmantel mit den drei nicht genutzten Zacken, sowie der Schlitz 63b im Hülsenmantel.

An Stelle der Spiral-, Uhren, oder Triebfeder kann das Federpaket auch einen Energiespeicher in Form einer anderen Torsions- oder Drehfeder aufweisen, beispielsweise eine aus Federstahl gewickelte Wendel- oder Helixfeder, in welche die zur Produktausschüttung notwendige potentielle Energie durch Torsion zweier Federenden geladen wird. Das Federpaket kann alternativ zu einem Autoinjektor mit nichtverstellbarer Dosis auch in einem als Autopen bezeichneten automatischen Injektionsgerät mit Dosiseinstellung eingesetzt werden.

Die eingangs genannte Patentanmeldung WO2016/205963 beschreibt ebenfalls ein Federpaket umfassend einen Federschaft mit distalem Endflansch, eine Spiralfeder, eine Federhülse und einen Federhülsendeckel, welcher in Abhängigkeit einer Spiralfederbreite an unterschiedlichen axialen Positionen am Federschaft lösbar befestigt werden kann. Der Federschaft weist eine axial ausgebildete Halterippe auf, in welcher das als Haltelasche ausgebildete innere Ende der Spiralfeder drehfest verankert werden kann, und die Federhülse weist eine axial orientierte Haltekante auf, an welcher das als Haltelasche ausgebildete äussere Ende der Spiralfeder drehfest verankert werden kann. Die Haltelasche umfasst eine Haltezunge welche bei einer Biegekante im Federband gegenüber der Federspannrichtung um mehr als 90° abgewinkelt ist und in Richtung zum inneren Ende der Spiralfeder weist. Zum Verankern wird die Haltelasche über die Haltekante gespannt, und unter Nachlassen der Federspannung so geführt, dass sie mit der Haltekante verhakt. Die Biegekante im Federband ist durch die nach der Verankerung erfolgende Verdrehung der Federhülse relativ zur Federachse zum Laden von potentieller Energie in die Spiralfeder noch stärker beansprucht.

Ein Federpaket für eine Injektionsvorrichtung nach dem Stand der Technik weist also folgende Elemente auf:
einen Federschaft mit einer Federachse, eine Torsions- oder Drehfeder, insbesondere eine aus einem Federband gewickelte Spiral- oder Triebfeder, und eine Federhülse, wobei die Torsionsfeder mit einem inneren Ende mit dem Federschaft und mit einem äusseren Ende mit der Federhülse jeweils bezüglich der Federachse drehfest verbunden ist;
ein erstes und ein zweites Sicherungselement welche an der Federhülse und am Federschaft fest angeordnet sind und in einem Eingriff die Federhülse relativ zum Federschaft bezüglich der Federachse drehfest sichern, wobei durch eine Freistellungsbewegung des ersten Sicherungselements der Eingriff gelöst werden kann;
ein Steuerelement welches bei der Montage des Federpakets auf eine Antriebseinheit der Injektionsvorrichtung durch ein Freigabeelement der Antriebseinheit so bewegt werden kann, dass dadurch das erste Sicherungselement eine Freistellungsbewegung ausführt und ein im Federpaket gespeichertes Drehmoment an die Antriebseinheit gekoppelt wird.

Das Federpaket kann vorteilhaft weitergebildet werden dadurch, dass
a) das äussere Ende der Torsionsfeder eine Schlaufe bildet oder eine Öffnung aufweist, welche unter Spannung der Torsionsfeder in eine Federspannrichtung, bevorzugt durch Ergreifen, Spannen und Führen des äusseren Endes des Spiralfederbandes, in einen Zacken oder Haken der Federhülse eingehängt werden kann;
b) der Zacken der Federhülse in die Federspannrichtung zeigt und durch eine Ausnehmung in einem Hülsenmantel der Federhülse gebildet wird;
c) das Spiralfederband im Bereich der Öffnung eine Stufe aufweist, bevorzugt mit einer Stufenhöhe zumindest annähernd gleich einer Dicke des Spiralfederbandes und des Hülsenmantels;
d) das erste Sicherungselement eine Freistellungsbewegung in Richtung der Federachse ausführt; und/oder
e) das Federband zwischen zwei unlösbar axialfest mit dem Federschaft verbundenen Endflanschen angeordnet ist.

Mit den genannten Federpaketen lässt sich eine modulare Federpaketfamilie definieren, umfassend ein erstes und ein zweites Federpaket, wobei das erste Federpaket umfasst einen ersten Federschaft mit zwei fest angebrachten Flanschen, eine erste Spiralfeder welche zwischen den zwei Flanschen um den Federschaft gewickelt ist und deren Breite dem Abstand der Flansche entspricht, und einer Federhülse, welche die Spiralfeder umgibt, und wobei das zweite Federpaket umfasst einen zweiten Federschaft mit zwei fest angebrachten Flanschen, eine zweite Spiralfeder welche zwischen den zwei Flanschen um den Federschaft gewickelt ist und deren Breite dem Abstand der Flansche entspricht, und einer Federhülse, welche die zweite Spiralfeder umgibt. Der erste und der zweite Federschaft sowie die Breite der ersten und der zweiten Spiralfeder sind verschieden, aber die Federhülse, und damit die Form und die Kopplungselemente zur Antriebseinheit, sind für beide Federpakete identisch. Federn mit unterschiedlichen Federbandbreiten lassen sich also in ein Verabreichungsgerät montieren ohne das dieses angepasst werden müsste. So kann bei gleichbleibender Konstruktion des Verabreichungsgerätes eine Anpassung der Kraft, welche beim Verabreichen eines bestimmten Medikaments wirken soll, stattfinden.

**BEZUGSZEICHENLISTE**

| | | | |
|---|---|---|---|
| 10 | Gehäuse | 30 | Abziehkappe |
| 10a | Sichtfenster | 31 | Schutzkappenentferner |
| 10b | Halteabschnitt | 40 | Nadelschutzhülse |
| 10c | Schnappelement | 40a | Hülsenarm |
| 10d | Gehäusekontur | 40b, c | Ausnehmung |
| 10e | Ausnehmung | 41 | Nadelschutzhülsenfeder |
| 11 | Spritzenhalter | 42 | Schalthülse |
| 11a | Auflageelement | 43 | Sperrhülse |
| 11b | Finger | 50 | Kolbenstange |
| 11c | Halterhülse | 50a | Ausnehmung |
| 11d, e, f | Schnappelement | 51 | Halteelement |
| 11g | Abstützelement | 51a | Haltearm |
| 12 | Endkappe | 51b, c | Eingriffselement |
| 12a | Öffnung | 51d | Abragung |
| 12b | Kappenkontur | 52 | Gewindestange |
| 12c | Haltenocken | 6 | Federpaket |
| 13 | Mechanikhalter | 61 | Federschaft |
| 13a | Haltefederabschnitt | 61a, c | Endflansch |
| 14 | Adapter | 61b, 61b' | Federachse |
| 14a | Auflageelement | 61d | Verlängerung |
| 14b | Finger | 61e | Anschlag |
| 14c | Adapterkörper | 61f' | Zwischenflansch |
| 15 | Adapterhalter | 62 | Spiralfeder |
| 15a | Halterhülse | 62a, 62a' | Öffnungen |
| 15b | Halteabschnitt | 63 | Federhülse |
| 15c, d, e | Schnappelement | 63a | Zacken |
| 2 | Fertigspritze | 63b | Schlitz |
| 21 | Spritzenkörper | 63c | Sperrschnapper |
| 22 | Spritzenschulter | 63d | Zahn |
| 23 | Stopfen | 63e | Steuerelement |
| 24 | Produktaufnahmeraum | 63f | Haltestruktur |
| 25 | Injektionsnadel | 63g | Stoppelement |
| 26 | Fingerflansch | 7 | Antriebseinheit |
| 27 | Nadelschutzkappe | 71 | Freigabeelement |
| | | 72 | Gegenanschlag |

## Patentansprüche

1. Modularer Spritzenhalter zum Halten einer Fertigspritze (2) in einem Injektionsgerät mit einem Gehäuse (10) und einer Längsachse, umfassend einen Adapterhalter (15) und einen Adapter (14) mit einem Adapterkörper (14c) und einem daran beweglich angebrachten Auflageelement (14a) für eine Spritzenschulter (22) der Fertigspritze, wobei
- der Adapter (14) ausgebildet ist zur Aufnahme der Fertigspritze (2) in Richtung der Längsachse unter Auslenkung des Auflageelements (14a) durch eine Nadelschutzkappe (27) der Fertigspritze,
- der Adapterhalter (15) ausgebildet ist zur Aufnahme des Adapters (14), so dass dadurch das Auflageelement (14a) von einem Halteabschnitt (15b) des Adapterhalters gegen ein radiales Auslenken blockiert wird.

2. Modularer Spritzenhalter nach Anspruch 1, **dadurch gekennzeichnet, dass** der Adapter (14) einen flexiblen Finger (14b) aufweist und das Auflageelement (14a) an einem distalen Ende des Fingers befestigt ist und in Richtung einer Rotation um die Längsachse vom Finger absteht.

3. Modularer Spritzenhalter nach Anspruch 1, **dadurch gekennzeichnet, dass** der Adapter (14) und der Adapterhalter (15) derart ausgebildet sind, dass das Auflageelement (14a) in axialer Richtung unmittelbar am Gehäuse (10) ansteht.

4. Modularer Spritzenhalter nach Anspruch 1, **dadurch gekennzeichnet, dass** der Adapter (14) und der Adapterhalter (15) derart ausgebildet sind, dass sie eine Rotation der Fertigspritze (2) um die Längsachse erlauben.

5. Modularer Spritzenhalter nach Anspruch 1, **dadurch gekennzeichnet, dass** der Adapter (14) zwei flexible Finger (14b) mit je einem Auflageelement (14a) zur Aufnahme von Axialkräften der Spritzenschulter (22) umfasst.

6. Verfahren zur Montage einer Fertigspritze (2) in ein Injektionsgerät mit einem Gehäuse (10) und einer Längsachse, umfassend
- Einsetzen eines Spritzenhalters (11) mit einer Halterhülse (11c) und einem daran beweglich angebrachten Auflageelement (11a) in ein Gehäuse (10) des Injektionsgeräts in eine erste axiale Position, in welcher das Auflageelement (11a) auslenkbar ist,
- Blockieren des Spritzenhalters (11) gegen eine Bewegung in distale Richtung,
- Einführen der Fertigspritze (2) in den Spritzenhalter (11) in distaler Richtung unter Auslenkung des Auflageelements (11a) durch eine Nadelschutzkappe (27) der Fertigspritze,
- Freigeben einer Bewegung des Spritzenhalters in distale Richtung, und
- Bewegen des Spritzenhalters (11) in eine zweite axiale Position, in welcher das Auflageelement (11a) durch einen Halteabschnitt (10b) des Gehäuses gegen ein radiales Auslenken blockiert ist.

7. Verfahren zur Montage einer Fertigspritze nach Anspruch 6, umfassend
- Blockieren des Spritzenhalters (11) gegen eine Bewegung in distale Richtung durch ein an ein Abstützelement (11g) des Spritzenhalters angreifendes Montagetool.

8. Verfahren zur Montage einer Fertigspritze nach Anspruch 7, umfassend
- Einführen des Montagetools senkrecht zur Längsachse durch ein Sichtfenster (10a) im Gehäuse.

9. Injektionsgerät mit einem modularen Spritzenhalter oder nach einem Verfahren gemäss einem der vorangegangenen Ansprüche, **dadurch gekennzeichnet, dass** das Injektionsgerät ein Autoinjektor ist, die Fertigspritze (2) einen Produktaufnahmeraum (24) und eine daran unlösbar befestigte Injektionsnadel (25) umfasst und axial unverschiebbar im Gehäuse (10) gehalten ist, und dass der Produktaufnahmeraum (24) ein nominales Füllvolumen von weniger als 2.25 ml aufweist.

10. Injektionsgerät nach Anspruch 9, **dadurch gekennzeichnet, dass** das Injektionsgerät einen Antrieb umfasst mit einer Spiralfeder (62), welche im Auslieferzustand mindestens die Energie für eine vollständige Ausschüttung des Produktes im Produktaufnahmeraum (24) speichert.

11. Injektionsgerät nach Anspruch 10, **dadurch gekennzeichnet, dass** der Antrieb ein Federpaket umfasst mit einem Federschaft (61), der Spiralfeder (62), und einer Federhülse (63),
- wobei die Spiralfeder mit einem inneren Ende mit dem Federschaft und mit einem äusseren Ende mit der Federhülse jeweils bezüglich der Längsachse drehfest verbunden ist;
- wobei ein erstes und ein zweites Sicherungselement (63c, 61e) welche an der Federhülse und am Federschaft fest angeordnet sind und in einem Eingriff die Federhülse relativ zum Federschaft bezüglich der Längsachse drehfest sichern durch eine Freistellungsbewegung des ersten Sicherungselements (63c) aus dem Eingriff gelöst werden können;
- wobei ein Steuerelement (63e) des Federpakets bei der Montage des Federpakets auf eine Antriebseinheit (7) der Injektionsvorrichtung durch ein Freigabeelement (71) der Antriebseinheit so bewegbar ist, dass dadurch das erste Sicherungselement (63c) eine Freistellungsbewegung ausführt und ein im Federpaket gespeichertes Drehmoment an die Antriebseinheit gekoppelt wird.

12. Injektionsgerät nach Anspruch 11, **dadurch gekennzeichnet, dass** das äussere Ende der Spiralfeder (62) eine Öffnung (62a) aufweist, welche unter Spannung der Spiralfeder in einen Zacken (63a) der Federhülse (63) eingehängt werden kann.
